# EUROPÄISCHE PATENTSCHRIFT

(11) **EP 0 664 826 B1**
(45) Veröffentlichungstag und Bekanntmachung des Hinweises auf die Patenterteilung: **19.06.1996**
(21) Anmeldenummer: 93922953.0
(22) Anmeldetag: 14.10.1993
(51) Int. Cl.: C09K 19/40, C08G 77/38

(54) **FLÜSSIGKRISTALLINE DORISTEROLHALTIGE ORGANOSILOXANE**
LIQUID CRYSTALLINE ORGANOSILOXANES CONTAINING DORISTEROL
ORGANOXILOXANES A CRISTAUX LIQUIDES CONTENANT DU DORISTEROL

(30) Priorität: 15.10.1992 DE 4234845
(43) Veröffentlichungstag der Anmeldung: 02.08.1995
(73) Patentinhaber: Consortium für elektrochemische Industrie GmbH, D-81379 München (DE)
(72) Erfinder: WEITZEL, Hans-Peter, D-84571 Reischach (DE); KREUZER, Franz-Heinrich, D-82152 Martinsried (DE); MAURER, Robert, D-81371 München (DE)
(74) Vertreter: Fritz, Helmut, Dr.
(86) Internationale Anmeldenummer: EP9302842
(87) Internationale Veröffentlichungsnummer: WO9409086

(56) Entgegenhaltungen:
- EP-A- 0 346 911
- EP-A- 0 358 208
- EP-A- 0 466 183
- US-A- 3 888 892
- US-A- 3 907 406
- MOLECULAR CRYSTALS AND LIQUID CRYSTALS BULLETIN Bd. 147 , Juni 1987 , READING GB Seiten 43 - 60 N.W.ADAMS 'synthesis and chromatographic properties of liquid crystalline polysiloxanes containing steroid substituents'
- LIQUID CRYSTALS Bd. 11, Nr. 4 , April 1992 , LONDON GB Seiten 569 - 580 J.M.GILLI 'enhanced smectic polymorphism of a cholesteric side chain co-organosiloxane......'
- LIQUID CRYSTALS Bd. 11, Nr. 4 , April 1992 , LONDON GB Seiten 569 - 580 J.M.GILLI 'enhanced smectic polymorphism of a cholesteric side chain co-oligosiloxane.......'

## Beschreibung

Die vorliegende Erfindung betrifft flüssigkristalline Doristerylreste enthaltende Organosiloxane, ein Verfahren zu deren Herstellung, deren Verwendung, zu flüssigkristalline Doristerylreste enthaltenden Organosiloxanen kondensierbare Organosilane und Mischungen von doristerolhaltigen Organosiloxanen mit anderen flüssigkristallinen Materialien.

Für einige optische Anwendungen von flüssigkristallinen Materialien, wie z.B. in Notch-Filtern, ist es notwendig, cholesterische Phasen mit rechts- und cholesterische Phasen mit linkshändiger Helix zu besitzen, um sowohl links- als auch rechtszirkular polarisiertes Licht reflektieren zu können.

Für linkshelikale Filter wird dabei häufig auf Cholesterinverbindungen, also Steroidverbindungen, zurückgegriffen, die außer der Chiralität eine ausreichende Mesogenität, d.h. eine ausreichende Tendenz zur Ausbildung flüssigkristalliner Phasen mitbringen, um eine stabile Mesophase zu erzeugen. Dazu eignen sich beispielsweise die aus der US-A 4,410,570 bekannten cyclischen Organosiloxane mit Cholesterylresten.

Bei der Darstellung rechtshelikaler Filter wurde bislang auf nichtsteroidale Moleküle zurückgegriffen, die diverse Nachteile besitzen.

Aus M.L.Tsai, S.H.Chen, S.D.Jacobs, Appl. Phys. Lett., 54, 2395, 1989, ist bekannt, daß modifizierte Hydroxypropylcellulose keine ausreichende Mesophasenstabilität besitzt und Poly-Benzyl-glutamate einen für die Langzeitstabilität zu tiefen Glaspunkt von -25°C aufweisen.

Das einzige bekannte Sterol, dessen Ester eine Rechtshelix liefern, ist das in US-A 3,907,406 und US-A 3,888,892 beschriebene Cholest-8(14)-en-3β-ol der Formel 1, 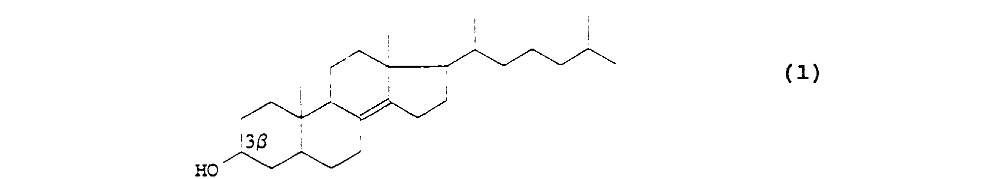 im folgenden kurz als Doristerol bezeichnet. Die beschriebenen Verbindungen sind aliphatische Ester, Halogen- oder Carbonatderivate von Doristerol, die zum einen nur geringe Mesophasenbreiten besitzen und zum anderen auf Grund des kristallinen Zustandes bei Raumtemperatur nur bei erhöhter Temperatur benutzt werden können.

Aufgabe der vorliegenden Erfindung war es, rechtshelikale flüssigkristalline Materialien bereitzustellen, die bei Raumtemperatur eine stabile cholesterische Phase besitzen, eine selektive Reflexion von rechtshändig polarisiertem Licht ermöglichen und deren Reflexionswellenlänge weitgehend temperaturunabhängig ist. Außerdem sollte dieses Material eine möglichst hohe helical twisting power (htp), d.h. eine hohe Tendenz zur Ausbildung von helikalen Schraubenstrukturen besitzen, um den Anteil der teuren chiralen Komponente niedrig wählen zu können.

Die vorstehend genannten Aufgaben werden durch die vorliegende Erfindung gelöst durch flüssigkristalline Organosiloxane, die dadurch gekennzeichnet sind, daß sie pro Molekül mindestens einen über die 3β-Position gebundenen Doristerylrest aufweisen.

Die erfindungsgemäßen flüssigkristallinen Organosiloxane zeigen eine geringe Neigung zur Kristallisation, eine Stabilisierung und Verbreiterung der cholesterischen Phase gegenüber den bekannten Doristerolderivaten und liegen bei Raumtemperatur im Glaszustand vor.

Die cholesterische Phase kann nach Orientierung oberhalb der Glastemperatur durch Abschrecken in den Glaszustand konserviert werden und ist bei Raumtemperatur stabil. Die höheren Glaspunkte der erfindungsgemäßen flüssigkristallinen Organosiloxane im Vergleich zu bekannten rechtshelikalen Materialien bewirken eine höhere Stabilität in der eingefrorenen cholesterischen Phase bei Raumtemperatur.

Die erfindungsgemäßen flüssigkristallinen Organosiloxane weisen deutlich breitere Mesophasen auf als die bekannten niedermolekularen Doristerylverbindungen. Beispielsweise liegen die Durchschnittswerte bei den erfindungsgemäßen flüssigkristallinen cyclischen Organosiloxanen bei etwa g30*130i, während beispielsweise Doristerylpropionat Werte von k80,5n*83,5i und Doristerylbenzoat Werte von k111n* 140,5i aufweisen.

Die erfindungsgemäßen flüssigkristallinen Organosiloxane weisen eine deutlich größere htp und damit ein besseres optisches Drehvermögen pro Doristerylrest auf, als die niedermolekularen Doristerylderivate, so daß zur Erzielung der gleichen optischen Wirkung geringere Mengen an Doristerolverbindung eingesetzt werden müssen. Die in der US-A-4,410,570 beschriebenen cyclischen Organosiloxane mit Cholesterylresten zeigen im Vergleich zu den entsprechenden niedermolekularen Cholesterinverbindungen keine größere htp.

Vorzugsweise enthalten die flüssigkristallinen Organosiloxane neben den Doristerylresten noch andere mesogene Reste, die die nachträgliche radikalische oder ionische Vernetzung des rechtshelikalen Filters ermöglichen.

Durch Verändern des Gehalts an Doristerylresten und des Verhältnisses von Doristerylresten zu anderen mesogenen Resten in den erfindungsgemäßen flüssigkristallinen Organosiloxanen kann die Reflexionswellenlänge der selektiven Reflexion eingestellt werden. Aufgrund der großen htp-Werte benötigt man nur einen Anteil von 10 bis 20 Mol-%, bezogen auf alle in den Organosiloxanen vorhandenen mesogenen Reste an Doristerylresten, um eine Reflexion im sichtbaren Bereich zu erhalten, während bei den entsprechenden cholesterinhaltigen Organosiloxanen zwischen 40 und 50 Mol-% Cholesterylreste benötigt werden.

Die Doristerylreste sind vorzugsweise über andere mesogene Gruppen an das Siloxangerüst gebunden. Der Begriff "mesogene Gruppen" ist in der Fachwelt gut bekannt. Es sind dies diejenigen Gruppen, die in einem Molekül flüssigkristalline Eigenschaften hervorrufen können.

Beispiele für mesogene Gruppen sind Derivate des Cyclohexans, wie Cyclohexylcarbonsäurecyclohexylester, Cyclohexylcarbonsäurephenylester, Cyclohexylbenzole, Dicyclohexylderivate, Derivate des Stilbens, Benzoesäurephenylester und seine Derivate, Steroide, wie Cholesterin, dessen Derivate, wie Cholesterinester, Cholestan und dessen Derivate, Benzylidenaniline, Azobenzol und seine Derivate, Azoxybenzol und dessen Derivate, Alkyl- und Alkoxyderivate von Biphenyl, Schiff'sche Basen.

Oftmals ist es aus anwendungstechnischen Gründen erwünscht, daß die mesogenen Gruppen polare Funktionen, wie beispielsweise die Nitrilgruppe, enthalten, um im Flüssigkristall einen hohen dielektrischen Anisotropieeffekt zu erzielen.

Bei den vorstehend genannten flüssigkristallinen Organosiloxanen handelt es sich vorzugsweise um solche, die aus mindestens zwei Einheiten der allgemeinen Formel 2,

[BₒRₚH_{q}SiO_{(4-o-p-q)/2}] (2),

aufgebaut sind,
in der **B** einen mesogenen Rest der allgemeinen Formel 3,

R¹-(X¹ ₐ-A¹ _{b}-A² _{c})_{d}-Zₑ-(-X² _{f}-A³ _{g}-A⁴ ₕ-)ᵢ-dor (3),

und gegebenenfalls einen mesogenen Rest der allgemeinen Formel 4,

R¹-(X¹ ₐ-A¹ _{b}-A² _{c})_{d}-Zₑ-(-X² _{f}-A³ _{g}-A⁴ ₕ-)ᵢ-A⁵ ₖ (4),

bedeutet, wobei in den vorstehenden Formeln 2, 3 und 4
- **R**: gleiche oder verschiedene, gegebenenfalls substituierte C₁-bis C₁₈-Kohlenwasserstoffreste,
- **o**: eine ganze Zahl im Wert von 0 bis 3,
- **p**: eine ganze Zahl im Wert von 0 bis 3 und einen durchschnittlichen Wert von 0,8 bis 2,2,
- **q**: eine ganze Zahl im Wert von 0 bis 3 und die Summe von **o, p** und **q** maximal 3 beträgt,
- **R**^{**1**}: einen Rest der Formel CₙHₘ bedeutet, in der
- **n**: eine ganze Zahl im Wert von 0 bis 20,
- **m**: den Wert von 2n besitzt, oder, falls n mindestens 2 bedeutet, auch den Wert von (2n-2) besitzen kann, und in R¹ eine oder mehrere Methyleneinheiten durch Sauerstoffatome ersetzt sein können, welche an Kohlenstoff- und/oder Siliciumatome gebunden sein können,
- **X**^{**1**} und **X**^{**2**}: gleiche oder verschiedene zweibindige Reste aus der Gruppe -O-, -COO-, -CONH-, -CO-, -S-, -C≡C-, -CH=CH-, -CH=N-, -CH₂CH₂-, -N=N- und -N=N(O)-,
- **A**^{**1**}**, A**^{**2**}**, A**^{**3**} und **A**^{**4**}: gleiche oder verschiedene zweibindige Reste, nämlich 1,4-Phenylen-, 1,4-Cyclohexylenreste, substituierte Arylene mit 1 bis 10 Kohlenstoffatomen, substituierte Cycloalkylene mit 1 bis 10 Kohlenstoffatomen und Heteroarylene mit 1 bis 10 Kohlenstoffatomen,
- **Z**: gleiche oder verschiedene zwei- bis vierwertige Benzol-, Cyclohexan- oder Cyclopentanreste,
- **dor**: einen über die 3β-Position gebundenen Doristerylrest bedeutet,
- **A**^{**5**}: gleiche oder verschiedene, gesättigte oder olefinisch ungesättigte Alkyl-, Alkoxy- oder Cycloalkylreste mit jeweils 1 bis 16 Kohlenstoffatomen, Cholestanreste, Cholesterylreste, Halogenatome, Wasserstoffatome, Hydroxyl-, Nitril-, Acryloxy-, (Meth)acryloxy-, (Meth)acryloxyethylenoxy-, (Meth)acryloxydi(ethylenoxy)-, (Meth)acryloxytri(ethylenoxy)- und Trialkylsiloxygruppen, deren Alkylreste jeweils 1 bis 8 Kohlenstoffatome besitzen,
- **a, b, c, d, f, g, h, i** und **k**: jeweils gleiche oder verschiedene ganze Zahlen im Wert von 0 bis 3, wobei die Summe **a+b+c+d+e+f+g+h+i+k** mindestens 2 und die Summe von **d** und **i** maximal 4 beträgt, und
- **e**: eine Zahl im Wert von 0 oder 1,
bedeuten.

Beispiele für unsubstituierte Reste **R** sind Alkylreste, wie der Methyl-, Ethyl-, n-Propyl-, iso-Propyl-, n-Butyl-, iso-Butyl-, tert.-Butyl-, n-Pentyl-, iso-Pentyl-, neo-Pentyl-, tert.-Pentylrest, Hexylreste, wie der n-Hexylrest, Heptylreste, wie der n-Heptylrest, Oktylreste, wie der n-Octylrest und iso-Oktylreste, wie der 2,2,4-Trimethylpentylrest, Nonylreste, wie der n-Nonylrest, Decylreste, wie der n-Decylrest, Dodecylreste, wie der n-Dodecylrest, Oktadecylreste, wie der n-Oktadecylrest; Alkenylreste, wie der Vinyl- und der Allylrest; Cycloalkylreste, wie Cyclopentyl-, Cyclohexyl-, Cycloheptylreste und Methylcyclohexylreste; Arylreste, wie der Phenyl-, Naphtyl-, Anthryl- und Phenanthrylreste; Alkarylreste, wie o-, m-, p-Tolylreste, Xylylreste und Ethylphenylreste; Aralkylreste, wie der Benzylrest, der α- und der β-Phenylethylrest;

Beispiele für substituierte Reste **R** sind Cyanalkylreste, wie der β-Cyanethylrest, und halogenierte Kohlenwasserstoffreste, beispielsweise Halogenalkylreste, wie der 3,3,3-Trifluor-n-propylrest, der 2,2,2,2',2',2'-Hexafluorisopropylrest, der Heptafluorisopropylrest, und Halogenarylreste, wie der o-, m-, und p-Chlorphenylrest.
Vorzugsweise bedeutet R jeweils einen gegebenenfalls halogenierten Kohlenwasserstoffrest mit 1 bis 18, insbesondere 1 bis 10, Kohlenstoffatomen.

Besonders bevorzugt als Reste R sind C₁- bis C₄-Alkylreste und Phenylreste, insbesondere Methylreste.

Die Reste X¹ und X² können, falls sie nicht symmetrisch gebaut sind, mit jedem ihrer Enden an jedem ihrer Bindungspartner verbunden sein. So kann beispielsweise in den vorstehenden Formeln 3 und 4 und in den nachstehenden Formeln der Rest -COO- auch als -OOC-, der Rest -CONH- auch als -NHCO-, -CH=N- auch als -N=CH- gebunden sein.

Als Substituenten für die substituierten Arylene und Cycloalkylene A¹, A², A³ und A⁴ sind Halogenatome, C₁- bis C₄-Alkoxyreste, Nitro- und Cyanogruppen, C₁- bis C₆-Alkylreste, Carboxy-(C₁- bis C₄-Alkyl)-Reste und Tri-(C₁- bis C₄-Alkyl)-Siloxyreste bevorzugt.

Vorzugsweise hat in R¹ n einen Wert von 3 bis 6 und vorzugsweise hat m den Wert 2n.

Beispiele für Reste A⁵ sind Alkylreste, wie der Methyl-, Ethyl-, n-Propyl-, iso-Propyl-, n-Butyl-, iso-Butyl-, tert.-Butyl-, n-Pentyl-, iso-Pentyl-, neo-Pentyl-, tert.-Pentylrest, Hexylreste, wie der n-Hexylrest, Heptylreste, wie der n-Heptylrest, Oktylreste, wie der n-Octylrest und iso-Oktylreste, wie der 2,2,4-Trimethylpentylrest, Nonylreste, wie der n-Nonylrest, Decylreste, wie der n-Decylrest, Dodecylreste, wie der n-Dodecylrest, Hexadecylreste, wie der n-Hexadecylrest; Alkenylreste, wie der Vinyl- und der Allylreste, Butenyl-, Pentenyl-, Hexenyl-, Heptenyl-, Oktenyl-, Oktadienyl-, Decenyl-, Dodecenyl- und Hexadecenylreste; Cycloalkylreste, wie Cyclopentyl-, Cyclohexyl-, Cycloheptylreste und Methylcyclohexylreste; Alkyloxyreste, wie der Methoxy-, Ethoxy-, n-Propoxy-, Isopropoxy-, n-, sec.- und tert.-Butoxyreste, Pentoxy-, Hexoxy-, Oktoxy-, Decoxy-, Hexadecoxyreste; Alkenoxyreste, wie der Allyloxyrest, Butenyloxy-, Pentenyloxy-, Hexenyloxy-, Oktenyloxy-, Decenyloxy- und Hexadecenyloxyreste; Cycloalkylreste, wie der Cyclopentyl-, Cyclohexyl-, Cycloheptylrest; Cycloalkenylreste, wie Cyclopentenyl-, Cyclohexenyl- und Cycloheptenylreste; Cholestanreste; der Cholesterylrest; Fluor-, Chlor- oder Bromatome; Wasserstoffatome; Hydroxyl-, Nitril- und Trimethylsilyl-, Triethylsilylgruppen.
Es ist ganz besonders bevorzugt, daß -R¹-(X¹ₐ-A¹_{b}-A²_{c})_{d}- in den vorstehenden Formeln 3 und 4 einen Rest der Formel 11, 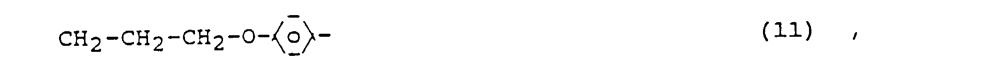 bedeutet.

Insbesondere bevorzugt als Reste der Formeln 3 und 4 sind diejenigen der allgemeinen Formeln 12 und 13, 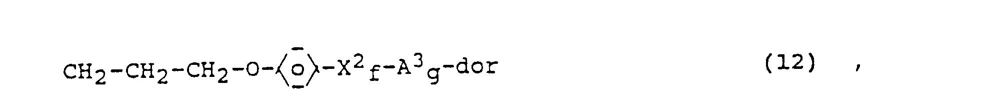 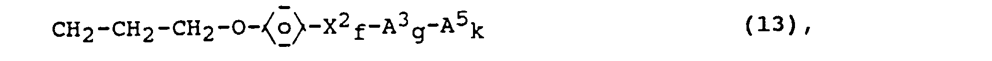 worin X², A³, A⁵, f, g und k die für Formeln 3 und 4 angegebenen Bedeutungen haben und vorzugsweise f den Wert 1, g entweder 0 oder 1 und k den Wert 1 besitzt.

Die erfindungsgemäßen flüssigkristallinen Organosiloxane können hergestellt werden durch Umsetzung von Organosiloxanen und/oder zu Organosiloxanen kondensierbaren Organosilanen mit mesogene Gruppen aufweisenden Alkenen oder Alkinen, wobei die Organosiloxane und mindestens ein Teil der Organosilane mindestens ein direkt an Silicium gebundenes Wasserstoffatom aufweist.

In einem bevorzugten Verfahren zur Herstellung von flüssigkristallinen Organosiloxanen der vorstehenden allgemeinen Formel 2, bei denen **n** in den mesogenen Resten der allgemeinen Formeln 3 und 4 eine ganze Zahl im Wert von 2 bis 20 bedeutet, werden Organosiloxane, die aus Einheiten der allgemeinen Formel 14,

[RₚH_{q+o}SiO_{(4-o-p-q)/2}] (14),

aufgebaut sind, und/oder Organosilane der allgemeinen Formel 15,

RᵣHₛ₊ₒSiY_{(4-o-r-s)/2} (15),

mit mesogenen Verbindungen der allgemeinen Formel 16,

R²-(X¹ ₐ-A¹ _{b}-A² _{c})_{d}-Zₑ-(-X² _{f}-A³ _{g}-A⁴ ₕ-)ᵢ-dor (16),

und gegebenenfalls mesogenen Verbindungen der allgemeinen Formel 17,

R²-(X¹ ₐ-A¹ _{b}-A² _{c})_{d}-Zₑ-(-X² _{f}-A³ _{g}-A⁴ ₕ-)ᵢ-A⁵ ₖ (17),

umgesetzt,
und, falls Organosilane der allgemeinen Formel 15 eingesetzt werden, die erhaltenen Organosilane der allgemeinen Formel 18,

BₒRᵣHₛSiY_{(4-o-r-s)/2} (18),

kondensiert,
wobei in den vorstehenden Formeln 14 bis 18
- **Y**: eine kondensierbare Gruppe,
- **R**^{**2**}: einen Rest der Formel CₙHₘ bedeutet, in der
- **m**: den Wert von 2n-1 oder 2n-3 besitzt,
- **r** und **s**: jeweils eine ganze Zahl im Wert von 0 bis 3 bedeuten, die Summe von **o, r** und **s** maximal 3 beträgt, und o, p, q, X¹, X², A¹, A², A³, A⁴, A⁵, a, b, c, d, e, f, g, h, i, k, Z, dor, B und R die für die allgemeinen Formeln 2, 3 und 4 angegebene Bedeutung haben.

Vorzugsweise bedeutet Y ein Halogenatom oder eine C₁- bis C₄-Alkoxygruppe, insbesondere ein Chloratom oder eine Methoxy- oder Ethoxygruppe. Vorzugsweise beträgt der Wert von s 0 oder 1.

In den vorstehenden allgemeinen Formeln 16 und 17 hat in R² n vorzugsweise einen Wert von 3 bis 6 und vorzugsweise hat m den Wert 2n-1.

Die Umsetzung von direkt an Silicium gebundene Wasserstoffatome aufweisenden Organosiloxanen und/oder zu Organosiloxanen kondensierbaren Organosilanen mit mesogene Gruppen aufweisenden Alkenen oder Alkinen erfolgt in an sich bekannter Weise, z.B. durch Hydrosilylierung in Lösungsmitteln, wie Kohlenwasserstoffen, Ethern oder Estern mit Metallen oder Verbindungen der Platingruppe als Katalysator. Geeignete Verfahren zur Hydrosilylierung sind beispielsweise in der EP-A-466 183 und in J.Küpfer, H.Finkelmann; Makromol.Chem., Rapid Commun.12,717, 1991, beschrieben.

Zur Herstellung von erfindungsgemäßen flüssigkristallinen Organosiloxanen, welche in den mesogenen Resten der allgemeinen Formel 4 Methacryloxy- und /oder Acryloxygruppen aufweisen, ist das in der EP-A-358 208 beschriebene Verfahren bevorzugt.

Vorzugsweise werden 0,1 bis 10 Mol, insbesondere 0,5 bis 2 Mol, von Verbindungen der Formeln 3 und 4 pro Grammatom direkt an Siliciumatome gebundene Wasserstoffatome bei der Hydrosilylierung eingesetzt.

Besonders bevorzugt als Siloxane der Formel (14) sind solche, die zu mindestens 90 % ihrer Einheiten aus solchen der Formeln 5 bis 10,
[(CH₃)₂SiO] **(5)**, [(CH₃)HSiO] **(6)**, [H₂SiO] **(7)**,
[H(CH₃)₂SiO_{1/2}] **(8)**, [(CH₃)₃SiO_{1/2}] **(9)** und [HSiO_{3/2}] **(10)**,
aufgebaut sind und 2 bis 100 Siliciumatome pro Molekül, insbesondere 2 bis 15 Siliciumatome pro Molekül, enthalten.

Werden im vorstehend geschilderten Verfahren Organosilane, beispielsweise der allgemeinen Formel 15, eingesetzt, so werden diese gemeinsam mit Doristerylresten enthaltenden Organosilanen oder Organosiloxanen nach an sich bekannten Verfahren zu flüssigkristallinen Organosiloxanen kondensiert. Dies kann u.a. durch Umsetzung mit Säuren, wie wäßriger Salzsäure, erfolgen. Derartige Verfahren sind beschrieben in W.Noll: Chemistry and Technology of Silicones, Academic Press, Orlando Fla., 1968, Seite 191 bis 239.

Durch die vorstehend beschriebenen Reaktionen erhält man ein Gemisch unterschiedlicher Moleküle.

Die neuen Organosilane der vorstehenden allgemeinen Formel 18, bei denen **o** eine ganze Zahl im Wert von 1, 2 oder 3 bedeutet, sind als Zwischenprodukte zur Herstellung der flüssigkristallinen Organosiloxane ebenfalls Gegenstand der vorliegenden Erfindung.

Die erfindungsgemäßen doristerolhaltigen Organosiloxane können auf verschiedene Weise in optischen Elementen und als polarisierende Farbfilter, insbesondere Notch-Filter, verwendet werden. Sie erlauben es, den rechtshändig polarisierten Anteil des Lichts in bestimmten vorgegebenen Spektralbereichen zu reflektieren.

Für die vorstehende Anwendung können sowohl Mischungen der erfindungsgemäßen Organosiloxane untereinander als auch Mischungen der erfindungsgemäßen Organosiloxane mit anderen flüssigkristallinen Materialien oder reine doristerolhaltige Organosiloxane genutzt werden. Insbesondere können auch Mischungen mit anderen flüssigkristallinen Substanzen, speziell auch mit linkshelikalen Materialien, verwendet werden, wodurch eine Abstimmung der Reflexionswellenlänge zwischen 400 nm rechtshelikal über infrarot rechtshelikal, nematisch (= unendlicher Pitch), infrarot linkshelikal bis 400 nm linkshelikal erfolgen kann.

Die Mischungen der flüssigkristallinen Organosiloxane untereinander und mit anderen flüssigkristallinen Materialien sind ebenfalls Gegenstand der vorliegenden Erfindung.

Die erfindungsgemäßen flüssigkristallinen Organosiloxane, welche in den mesogenen Resten der allgemeinen Formel 4 Methacryloxy- und/oder Acryloxygruppen aufweisen, können dreidimensional vernetzt werden. Diese Vernetzung wird vorzugsweise mittels freier Radikale bewirkt, welche durch Peroxide, durch UV-Licht oder durch energiereichere elektromagnetische Strahlung als UV-Licht, oder thermisch erzeugt werden. Die Vernetzung kann aber auch mittels direkt an Siliciumatome gebundene Wasserstoffatome enthaltende Vernetzer unter Katalyse durch die oben genannten Platinmetallkatalysatoren bewirkt werden. Sie kann auch kationisch oder anionisch erfolgen. Besonders bevorzugt ist die Vernetzung durch UV-Licht. Diese Vernetzung ist in der EP-A-358 208 beschrieben.

In den nachfolgenden Beispielen sind, falls jeweils nicht anders angegeben,
a) alle Mengenangaben auf das Gewicht bezogen;
b) alle Drücke 0,10 MPa (abs.);
c) alle Temperaturen 20°C;
d) htp = helical twisting power;
e) n* = cholesterisch;
f) g = glasartig;
g) k = kristallin;
h) i = isotrop.

### Beispiel 1

### a) Darstellung von 4-(Propen-2-oxy)benzoesäuredoristerylester

50 g Cholestadien wurden in 800 ml trockenem Essigsäureethylester, dem zuvor 30 ml Essigsäure zugesetzt wurden, gelöst und nach Zugabe von 1,5 g Platinoxid in einen Autoklaven von 2 L Volumen überführt. Die Hydrierung erfolgte bei 50°C und 10 atm Wasserstoffdruck und war nach 24 h beendet. Nach beendeter Reaktion wurde der Katalysator abfiltriert, das Lösungsmittel abrotiert und das erhaltene Doristerol aus Ethanol umkristallisiert. Ausbeute: 42,5 g (88,5 %). Die Reinheit der Substanz wurde mit Hilfe von ¹H-NMR- und ¹³C-NMR-Spektroskopie sichergestellt. 17 g 4-(Propen-2-oxy)benzoesäurechlorid und 32 g Doristerol wurden in 200 ml trockenem Toluol gelöst und 15 h zum Rückfluß erhitzt. Anschließend wurde das Lösungsmittel abdestilliert und der Rückstand aus Ethanol umkristallisiert; Ausbeute: 44,9 g (99 %), Smp.92°C (n*105i).

### b) Hydrosilylierung

1 g 4-(Propen-2-oxy)benzoesäuredoristerylester, 1,53 g 4-(Propen-2-oxy)benzoesäure-4'-phenylphenylester und 646 mg Pentamethylcyclopentasiloxan wurden in 20 ml trockenem Toluol gelöst und nach Zugabe von 0,1 ml einer Lösung von Dicyclopentadienplatindichlorid (1 Gew.-% in Methylenchlorid) 1 h auf 100°C erwärmt. Zu der auf 50°C abgekühlten Lösung gab man 1,45 g 4-(Propen-2-oxy)benzoesäure-(4-methacryloxy)phenylester, 500 ppm Hydrochinon und weitere 0,1 ml der Katalysatorlösung; diese Lösung wurde eine halbe Stunde bei 70-80°C gerührt. Nach beendeter Reaktion wurde der Katalysator über eine kurze, mit Kieselgel gefüllte Säule (1 = 3 cm, Durchmesser = 3 cm) abgetrennt und das Produkt in Ethanol ausgefällt. Man erhielt 2,8 g (60 %) einer Substanz mit einer Reflexionswellenlänge von 456 nm - entsprechend einer htp von 15,7 µm⁻¹. Die Substanz besaß einen Glaspunkt bei 27°C und einen Klärpunkt bei 126°C.

### Beispiel 2

1 g 4-(Propen-2-oxy)benzoesäuredoristerylester, 1,81 g 4-(Propen-2-oxy)benzoesäure-4'-phenylphenylester und 733 mg Pentamethylcyclopentasiloxan wurden in 20 ml trockenem Toluol gelöst und nach Zugabe von 0,1 ml einer Lösung von Dicyclopentadienplatindichlorid (1 Gew.-% in Methylenchlorid) 1 h auf 100°C erwärmt. Zu der auf 50°C abgekühlten Lösung gab man 1,65 g 4-(Propen-2-oxy)benzoesäure-(4-methacryloxy)phenylester, 500 ppm Hydrochinon und weitere 0,1 ml der Katalysatorlösung; diese Lösung wurde eine halbe Stunde bei 70-80°C gerührt. Nach beendeter Reaktion wurde der Katalysator über eine kurze mit Kieselgel gefüllte Säule (1 = 3 cm, Durchmesser = 3 cm) abgetrennt und das Produkt in Ethanol ausgefällt. Man erhielt 2,4 g (46 %) einer Substanz mit einer Reflexionswellenlänge von 500 nm - entsprechend einer htp von 16,1 µm⁻¹. Die Substanz besaß einen Glaspunkt bei 31°C und einen Klärpunkt bei 136°C.

### Beispiel 3

1 g 4-(Propen-2-oxy)benzoesäuredoristerylester, 2,69 g 4-(Propen-2-oxy)benzoesäure-4'-phenylphenylester und 1 g Pentamethylcyclopentasiloxan wurden in 20 ml trockenem Toluol gelöst und nach Zugabe von 0,1 ml einer Lösung von Dicyclopentadienplatindichlorid (1 Gew.-% in Methylenchlorid) 1 h auf 100°C erwärmt. Zu der auf 50°C abgekühlten Lösung gab man 2,25 g 4-(Propen-2-oxy)benzoesäure-(4-methacryloxy)phenylester, 500 ppm Hydrochinon und weitere 0,1 ml der Katalysatorlösung; diese Lösung wurde eine halbe Stunde bei 70-80°C gerührt. Nach beendeter Reaktion wurde der Katalysator über eine kurze mit Kieselgel gefüllte Säule (1 = 3 cm, Durchmesser = 3 cm) abgetrennt und das Produkt in Ethanol ausgefällt. Man erhielt 3,8 g (55 %) einer Substanz mit einer Reflexionswellenlänge von 681 nm - entsprechend einer htp von 15,8 µm⁻¹. Die Substanz besitzt einen Glaspunkt bei 23°C und einen Klärpunkt bei 122°C.

### Beispiel 4

### a) 2-(Propen-2-oxy)-6-naphthalincarbonsäuredoristerylester

3,2 g 2-(Propen-2-oxy)-6-naphthalincarbonsäurechlorid und 5 g Doristerol wurden in 40 ml trockenem Toluol gelöst und 6 h zum Rückfluß erhitzt. Das Rohprodukt wurde einrotiert und aus Ethanol umkristallisiert; Ausbeute 5,6 g (73 %), Smp.105°C(n*132i).

### b) Hydrosilylierung

2 g 2-(Propen-2-oxy)-6-naphthalincarbonsäuredoristerylester, 2,22 g 4-(Propen-2-oxy)benzoesäure-4'-phenylphenylester, und 1 g Pentamethylcyclopentasiloxan wurden in 20 ml trockenem Toluol gelöst und nach Zugabe von 0,1 ml einer Lösung von Dicyclopentadienplatindichlorid (1 Gew.-% in Methylenchlorid) 1 h auf 100°C erwärmt. Zu der auf 50°C abgekühlten Lösung gibt man 2,27 g 4-(Propen-2-oxy)benzoesäure-(4-methacryloxy)phenylester, 500 ppm Hydrochinon und weitere 0,1 ml der Katalysatorlösung; diese Lösung wurde eine halbe Stunde bei 70-80°C gerührt. Nach beendeter Reaktion wurde der Katalysator über eine kurze mit Kieselgel gefüllte Säule (1 = 3 cm, Durchmesser = 3 cm) abgetrennt und das Produkt in Ethanol ausgefällt. Man erhielt 4,9 g (65 %) einer Substanz mit einer Reflexionswellenlänge von 522 nm - entsprechend einer htp von 13,4 µm⁻¹. Die Substanz besaß einen Glaspunkt bei 53°C und einen Klärpunkt bei 147°C.

### Beispiel 5

Das gemäß Beispiel 1 hergestellte doristerolhaltige Organosiloxan mit einer Reflexionswellenlänge von 456 nm wurde mit einem entsprechenden cholesterinhaltigen Organosiloxan mit einer Reflexionswellenlänge von 555 nm in bestimmten Gewichtsverhältnissen gemischt. Die Gewichtsanteile und Reflexionswellenlängen der Mischungen sind der folgenden Tabelle zu entnehmen.

**Tabelle 1**

| Doristerolhaltiges Siloxan / Gew.-% | Cholesterinhaltiges Siloxan / Gew.-% | Reflexionswellenlänge / nm | Händigkeit der Helix r/l |
|---|---|---|---|
| 100 | 0 | 456 | r |
| 96,1 | 3,9 | 506 | r |
| 88,8 | 11,2 | 628 | r |
| 77,6 | 22,4 | 884 | r |
| 64,3 | 35,7 | 1891 | r |
| 34,5 | 65,5 | 1310 | l |
| 21,9 | 78,1 | 838 | l |
| 13,1 | 86,9 | 683 | l |
| 4,1 | 95,9 | 583 | l |
| 0 | 100 | 555 | l |

### Vergleichsbeispiel 6

Durch Zumischen in Merck-Nematen® ZLI-1565 und Vermessen der Grandjean-Cano-Disklinationslinien in einer Keilzelle wurden die htp-Werte von niedermolekularen Doristerolverbindungen vermessen. Doristerol besitzt eine htp von 8-9 µm⁻¹, 4-(Propen-2-oxy)benzoesäuredoristerylester eine htp von 5 µm⁻¹.

## Patentansprüche

1. Flüssigkristalline Organosiloxane, **dadurch gekennzeichnet**, daß sie pro Molekül mindestens einen über die 3β-Position gebundenen Doristerylrest aufweisen.

2. Flüssigkristalline Organosiloxane nach Anspruch 1, **dadurch gekennzeichnet**, daß sie aus mindestens zwei Einheiten der allgemeinen Formel 2,
[BₒRₚH_{q}Sio_{(4-o-p-q)/2}] (2),
aufgebaut sind,
in der **B** einen mesogenen Rest der allgemeinen Formel 3,
R¹-(X¹ ₐ-A¹ _{b}-A² _{c})_{d}-Zₑ-(-X² _{f}-A³ _{g}-A⁴ ₕ-)ᵢ-dor (3),
und gegebenenfalls einen mesogenen Rest der allgemeinen Formel 4,
R¹-(X¹ ₐ-A¹ _{b}-A² _{c})_{d}-Zₑ-(-X² _{f}-A³ _{g}-A⁴ ₕ-)ᵢ-A⁵ ₖ (4),
bedeutet, wobei in den vorstehenden Formeln 2, 3 und 4
**R** gleiche oder verschiedene, gegebenenfalls substituierte C₁- bis C₁₈-Kohlenwasserstoffreste,
**o** eine ganze Zahl im Wert von 0 bis 3,
**p** eine ganze Zahl im Wert von 0 bis 3 und einen durchschnittlichen Wert von 0,8 bis 2,2,
**q** eine ganze Zahl im Wert von 0 bis 3 und die Summe von **o, p** und **q** maximal 3 beträgt,
**R**^{**1**} einen Rest der Formel CₙHₘ bedeutet, in der
**n** eine ganze Zahl im Wert von 0 bis 20,
**m** den Wert von 2n besitzt, oder, falls n mindestens 2 bedeutet, auch den Wert von (2n-2) besitzen kann, und in R¹ eine oder mehrere Methyleneinheiten durch Sauerstoffatome ersetzt sein können, welche an Kohlenstoff- und/oder Siliciumatome gebunden sein können,
**X**^{**1**} und **X**^{**2**} gleiche oder verschiedene zweibindige Reste aus der Gruppe -O-, -COO-, -CONH-, -CO-, -S-, -C≡C-, -CH=CH-, -CH=N-, -CH₂CH₂-, -N=N- und -N=N(O)-,
**A**^{**1**}**, A**^{**2**}**, A**^{**3**} und **A**^{**4**} gleiche oder verschiedene zweibindige Reste, nämlich 1,4-Phenylen-, 1,4-Cyclohexylenreste, substituierte Arylene mit 1 bis 10 Kohlenstoffatomen, substituierte Cycloalkylene mit 1 bis 10 Kohlenstoffatomen und Heteroarylene mit 1 bis 10 Kohlenstoffatomen,
**Z** gleiche oder verschiedene zwei- bis vierwertige Benzol-, Cyclohexan- oder Cyclopentanreste,
**dor** ein über die 3β-Position gebundener Doristerylrest bedeutet,
**A**^{**5**} gleiche oder verschiedene, gesättigte oder olefinisch ungesättigte Alkyl-, Alkoxy- oder Cycloalkylreste mit jeweils 1 bis 16 Kohlenstoffatomen, Cholestanreste, Cholesterylreste, Halogenatome, Wasserstoffatome, Hydroxyl-, Nitril-, Acryloxy-, (Meth)acryloxy-, (Meth)acryloxyethylenoxy-, (Meth)acryloxydi(ethylenoxy)-, (Meth)acryloxytri(ethylenoxy)- und Trialkylsiloxygruppen, deren Alkylreste jeweils 1 bis 8 Kohlenstoffatome besitzen,
**a, b, c, d, f, g, h, i** und **k** jeweils gleiche oder verschiedene ganze Zahlen im Wert von 0 bis 3, wobei die Summe **a+b+c+d+e+f+g+h+i+k** mindestens 2 und die Summe von **d** und **i** maximal 4 beträgt, und
**e** eine Zahl im Wert von 0 oder 1 bedeuten.

3. Verfahren zur Herstellung von flüssigkristallinen Organosiloxanen gemäß Anspruch 2, bei denen **n** in den mesogenen Resten der allgemeinen Formeln 3 und 4 eine ganze Zahl im Wert von 2 bis 20 bedeutet, **dadurch gekennzeichnet**, daß Organosiloxane, die aus Einheiten der allgemeinen Formel 14,
[RₚH_{q+o}SiO_{(4-o-p-q)/2}] (14),
aufgebaut sind, und/oder Organosilane der allgemeinen Formel 15
RᵣHₛ₊ₒSiY_{(4-o-r-s)/2} (15),
mit mesogenen Verbindungen der allgemeinen Formel 16,
R²-(X¹ ₐ-A¹ _{b}-A² _{c})_{d}-Zₑ-(-X² _{f}-A³ _{g}-A⁴ ₕ-)ᵢ-dor (16),
und gegebenenfalls mesogenen Verbindungen der allgemeinen Formel 17,
R²-(X¹ ₐ-A¹ _{b}-A² _{c})_{d}-Zₑ-(-X² _{f}-A³ _{g}-A⁴ ₕ-)ᵢ-A⁵ ₖ (17),
umgesetzt werden,
und, falls Organosilane der allgemeinen Formel 15 eingesetzt werden, die erhaltenen Organosilane der allgemeinen Formel 18,
BₒRᵣHₛSiY_{(4-o-r-s)/2} (18),
kondensiert werden,
wobei in den vorstehenden Formeln 14 bis 18
**Y** eine kondensierbare Gruppe,
**R**^{**2**} einen Rest der Formel CₙHₘ bedeutet, in der
**m** den Wert von 2n-1 oder 2n-3 besitzt,
**r** und **s** jeweils eine ganze Zahl im Wert von 0 bis 3 bedeuten, die Summe von **o, r** und **s** maximal 3 beträgt, und o, p, q, X¹, X², A¹, A², A³, A⁴, A⁵, a, b, c, d, e, f, g, h, i, k, Z, dor, B und R die in Anspruch 2 angegebene Bedeutung haben.

4. Mischungen der flüssigkristallinen Organosiloxane gemäß Anspruch 1 oder 2 untereinander und mit anderen flüssigkristallinen Materialien, insbesondere linkshelikalen cholesterischen Materialien.

5. Verwendung der flüssigkristallinen Organosiloxane gemäß Anspruch 1 oder 2 oder der Mischungen gemäß Anspruch 4 als rechtshändige Filtermaterialien und in optischen Elementen.

6. Organosilane der allgemeinen Formel 18 gemäß Anspruch 3, bei denen o eine ganze Zahl im Wert von 1, 2 oder 3 bedeutet.

## Claims

1. Liquid crystalline organosiloxanes, characterized in that they contain at least one doristeryl radical bonded via the 3β-position per molecule.

2. Liquid crystalline organosiloxanes according to Claim 1, characterized in that they are constructed from at least two units of the general formula 2
[BₒRₚH_{q}SiO_{(4-o-p-q)/2}] (2),
in which **B** is a mesogenic radical of the general formula 3
R¹-(X¹ ₐ-A¹ _{b}-A² _{c})_{d}-Zₑ-(-X² _{f}-A³ _{g}-A⁴ ₕ-)ᵢ-dor (3),
and optionally a mesogenic radical of the general formula 4
R¹-(X¹ ₐ-A¹ _{b}-A² _{c})_{d}-Zₑ-(-X² _{f}-A³ _{g}-A⁴ ₕ-)ᵢ-A⁵ ₖ (4),
where in the above formulae 2, 3 and 4
**R** is identical or different, optionally substituted C₁- to C₁₈-hydrocarbon radicals,
**o** is an integer of value 0 to 3,
**p** is an integer of value 0 to 3 and an average value of 0.8 to 2.2,
**q** is an integer of value 0 to 3 and the sum of **o, p** and **q** is at most 3,
**R**^{**1**} is a radical of the formula CₙHₘ in which
**n** is an integer of value 0 to 20,
**m** has the value 2n, or if n is at least 2, can also have the value (2n-2), and in R¹ one or more methylene units can be replaced by oxygen atoms which can be bonded to carbon and/or silicon atoms,
**X**^{**1**} and **X**^{**2**} are identical or different bivalent radicals from the group consisting of -O-, -COO-, -CONH-,-CO-, -S-, -C≡C-, -CH=CH-, -CH=N-, -CH₂CH₂-, -N=N- and -N=N(O)-,
**A**^{**1**}**, A**^{**2**}**, A**^{**3**} and **A**^{**4**} are identical or different bivalent radicals, namely 1,4-phenylene or 1,4-cyclohexylene radicals, substituted arylenes having 1 to 10 carbon atoms, substituted cycloalkylenes having 1 to 10 carbon atoms and heteroarylenes having 1 to 10 carbon atoms,
**Z** is identical or different bi- or tetravalent benzene, cyclohexane or cyclopentane radicals,
**dor** is a doristeryl radical bonded via the 3β-position,
**A**^{**5**} is identical or different, saturated or olefinically unsaturated alkyl, alkoxy or cycloalkyl radicals in each case having 1 to 16 carbon atoms, cholestane radicals, cholesteryl radicals, halogen atoms, hydrogen atoms, hydroxyl, nitrile, acryloxy, (meth)acryloxy, (meth)acryloxyethylenoxy, (meth)acryloxydi(ethylenoxy), (meth)acryloxytri(ethylenoxy) and trialkylsiloxy groups whose alkyl radicals in each case have 1 to 8 carbon atoms,
**a, b, c, d, f, g, h, i** and **k** in each case are identical or different integers of value 0 to 3, the sum **a+b+c+d+e+f+g+h+i+k** being at least 2 and the sum of d and i being at most 4, and
**e** is an integer of value 0 or 1.

3. Process for the preparation of liquid crystalline organosiloxanes according to Claim 2, in which **n** in the mesogenic radicals of the general formulae 3 and 4 is an integer of value 2 to 20, characterized in that organosiloxanes which are constructed from units of the general formula 14
[RₚH_{q+o}SiO_{(4-o-p-q)/2}] (14)
and/or organosilanes of the general formula 15
RᵣHₛ₊ₒSiY_{(4-o-r-s)/2} (15)
are reacted with mesogenic compounds of the general formula 16
R²-(X¹ ₐ-A¹ _{b}-A² _{c})_{d}-Zₑ-(-X² _{f}-A³ _{g}-A⁴ ₕ-)ᵢ-dor (16)
and optionally mesogenic compounds of the general formula 17
R²-(X¹ ₐ-A¹ _{b}-A² _{c})_{d}-Zₑ-(-X² _{f}-A³ _{g}-A⁴ ₕ-)ᵢ-A⁵ ₖ (17)
and, if organosilanes of the general formula 15 are employed, the organosilanes obtained of the general formula 18
BₒRᵣHₛSiY_{(4-o-r-s)/2} (18)
are condensed,
where in the above formulae 14 to 18
**Y** is a condensable group,
**R**^{**2**} is a radical of the formula CₙHₘ, in which
**m** has the value 2n-1 or 2n-3,
**r** and **s** in each case are an integer of value 0 to 3, the sum of **o, r** and **s** is at most 3, and o, p, q, X¹, X², A¹, A², A³, A⁴, A⁵, a, b, c, d, e, f, g, h, i, k, Z, dor, B and R have the meaning indicated in Claim 2.

4. Mixtures of the liquid crystalline organosiloxanes according to Claim 1 or 2 with one another and with other liquid crystalline materials, in particular left-handed helical cholesteric materials.

5. Use of the liquid crystalline organosiloxanes according to Claim 1 or 2 or the mixtures according to Claim 4 as right-handed filter materials and in optical elements.

6. Organosilanes of the general formula 18 according to Claim 3, in which o is an integer of value 1, 2 or 3.

## Revendications

1. Organosiloxanes à cristaux liquides, caractérisés en ce qu'ils présentent par molécule au moins un résidu doristéryle lié par l'intermédiaire de la position 3-β.

2. Organosiloxanes à cristaux liquides selon la revendication 1, caractérisés en ce qu'ils sont constitués à partir d'au moins deux unités de formule générale 2,
[BₒRₚH_{q}SiO_{(4-o-p-q)/2}] (2),
dans laquelle B désigne un résidu mésogène de formule générale 3,
R¹-(X¹ ₐ-A¹ _{b}-A² _{c})_{d}-Zₑ-(-X² _{f}-A³ _{g}-A⁴ ₕ-)ᵢ-dor (3),
et, le cas échéant, un résidu mésogène de formule générale 4,
R¹-(X¹ ₐ-A¹ _{b}-A² _{c})_{d}-Zₑ-(-X² _{f}-A³ _{g}-A⁴ ₕ-)ᵢ-A⁵ ₖ (4),
dans les formules précédentes 2, 3 et 4,
R représentant des résidus d'hydrocarbures en C₁ à C₁₈, identiques ou différents, le cas échéant substitués,
o représentant un nombre entier d'une valeur de 0 à 3,
p représentant un nombre entier d'une valeur de 0 à 3 et une valeur moyenne de 0,8 à 2,2,
q représentant un nombre entier d'une valeur de 0 à 3, et la somme de o, p et q étant au maximum de 3,
R¹ désignant un résidu de formule CₙHₘ, dans laquelle
n représente un nombre entier d'une valeur de 0 à 20,
m possède la valeur de 2n, ou, dans le cas où n représente au moins 2, m peut aussi posséder la valeur de (2n-2), et dans R¹, une ou plusieurs unités de méthylène pouvant être remplacées par des atomes d'oxygène, qui peuvent être liés à des atomes de carbone et/ou de silicium,
X¹ et X² représentant des résidus divalents, identiques ou différents, du groupe -O-, -COO-, -CONH-, -CO-, -S-, -C≡C-, -CH=CH-, -CH=N-, -CH₂CH₂-, -N=N- et -N=N(O)-,
A¹, A², A³ et A⁴ représentant des résidus divalents, identiques ou différents, à savoir les résidus 1,4-phénylène, 1,4-cyclohexylène, des arylènes substitués ayant de 1 à 10 atomes de carbone, des cycloalkylènes substitués ayant de 1 à 10 atomes de carbone et des hétéro-arylènes ayant de 1 à 10 atomes de carbone,
Z représentant des résidus de benzène, de cyclohexane ou de cyclopentane, divalents à tétravalents, identiques ou différents,
dor représentant un résidu doristéryle lié par l'intermédiaire de la position 3-β,
A⁵ représentant des résidus alkyles, alkoxy, ou cycloalkyles, saturés ou oléfiniquement insaturés, identiques ou différents, ayant à chaque fois de 1 à 16 atomes de carbone, des résidus de cholestanol, des résidus cholestéryle, des atomes d'halogène, des atomes d'hydrogène, des groupements hydroxyle, nitrile, acroyle, (méth)acroyle, (méth) acroyléthylènoxy, (méth)acroyldi-(éthylènoxy), (méth)acroyltri(éthylènoxy), et trialkylsiloxy, dont les résidus alkyles possèdent à chaque fois de 1 à 8 atomes de carbone,
a, b, c, d, f, g, h, i et k représentant à chaque fois des nombres entiers identiques ou différents d'une valeur de 0 à 3, la somme a+b+c+d+e+f+g+h+i+k étant au moins de 2, et la somme de d et de i étant au maximum de 4 et
e représentant un nombre ayant la valeur de 0 ou 1.

3. Procédé de fabrication d'organosiloxanes à cristaux liquides selon la revendication 2, pour lesquels n représente, dans les résidus mésogènes des formules générales 3 et 4, un nombre entier d'une valeur de 2 à 20, caractérisé en ce que l'on fait réagir des organosiloxanes, qui sont constitués à partir des unités de formule générale 14,
[RₚH_{q+o}SiO_{(4-o-p-q)/2}] (14)
et/ou des organosiloxanes de formule générale 15
RᵣHₛ₊ₒSiY_{(4-o-r-s)/2} (15),
avec des composés mésogènes de formule générale 16,
R²-(X¹ ₐ-A¹ _{b}-A² _{c})_{d}-Zₑ-(X² _{f}-A³ _{g}-A⁴ ₕ-)ᵢ-dor (16),
et, le cas échéant, des composés mésogènes de formule générale 17,
R²-(X¹ ₐ-A¹ _{b}-A² _{c})_{d}-Zₑ-(-X² _{f}-A³ _{g}-A⁴ ₕ-)ᵢ-A⁵ ₖ (17),
et, dans le cas où des organosilanes de formule générale 15 sont utilisés, en ce que l'on procède à la condensation des organosilanes obtenus de formule générale 18,
BₒRᵣSiY_{(4-o-r-s)/2} (18),
dans les formules précédentes 14 à 18,
Y représentant un groupement capable de condensation,
R₂ représentant un résidu de formule CₙHₘ, dans lequel
m possède la valeur de 2n-1 ou de 2n-3,
r et s représentant à chaque fois un nombre entier d'une valeur de 0 à 3, la somme de o, r et s étant au maximum de 3, et o, p, q, X¹, X², A¹, A², A³, A⁴, A⁵, a, b, c, d, e, f, g, h, i, k, Z, dor, B et R ayant les significations indiquées dans la revendication 2.

4. Mélanges des organosiloxanes à cristaux liquides selon la revendication 1 ou 2 les uns avec les autres et avec d'autres matériaux à cristaux liquides, en particulier des matériaux cholestériques à hélicoïde gauche.

5. Utilisation des organosiloxanes à cristaux liquides selon la revendication 1 ou 2, ou des mélanges selon la revendication 4 en tant que matériaux de filtre à rotation vers la droite, et dans des éléments optiques.

6. Organosiloxanes de formule générale 18 selon la revendication 3, pour lesquels o représente un nombre entier ayant la valeur de 1, 2, ou 3.
